(19) Europäisches Patentamt
European Patent Office
Office européen des brevets

(11) Veröffentlichungsnummer: **0 019 103**
**B1**

# (12) EUROPÄISCHE PATENTSCHRIFT

(45) Veröffentlichungstag der Patentschrift: **22.12.82**

(51) Int. Cl.³: **C 08 G 59/16**, C 07 D 303/02, C 08 K 5/15, C 08 L 23/02

(21) Anmeldenummer: **80102149.4**

(22) Anmeldetag: **22.04.80**

(54) **Phenolgruppen enthaltende Epoxidharze, ihre Verwendung und sie enthaltende Kunststoffmassen.**

(30) Priorität: **26.04.79 DE 2916877**

(43) Veröffentlichungstag der Anmeldung: **26.11.80 Patentblatt 80/24**

(45) Bekanntmachung des Hinweises auf die Patenterteilung: **22.12.82 Patentblatt 82/51**

(84) Benannte Vertragsstaaten: **BE CH DE FR GB LI NL**

(56) Entgegenhaltungen:
**DE-A-1 745 035**
**US-A-4 130 549**

**SPRINGER-VERLAG, Berlin-Heidelberg-New York, 1966, J. VOIGT „Die Stabilisierung der Kunststoffe gegen Licht und Wärme"**

(73) Patentinhaber: **HOECHST AKTIENGESELLSCHAFT, Postfach 80 03 20, D-6230 Frankfurt/Main 80 (DE)**

(72) Erfinder: **Mayer, Norbert, Dr., Ziegelgrundweg 17, D-8901 Gablingen (DE)**
Erfinder: **Pfahler, Gerhard, Dr., Karlsbader Strasse 27, D-8900 Augsburg (DE)**
Erfinder: **Scheidl, Franz, Dr., Hans-Fischer-Strasse 4, D-8906 Gersthofen (DE)**

**EP 0 019 103 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Phenolgruppen enthaltende Epoxidharze, ihre Verwendung und sie enthaltende Kunststoffmassen

Bekanntlich werden Kunststoffen, insbesondere Polyolefinen, seit langem phenolische Antioxidantien zugesetzt, um ihren Abbau unter dem Einfluss von atmosphärischem Sauerstoff und Wärme hinauszuzögern. Die Wirkungsdauer der Langzeitstabilisierung wird u.a. jedoch dadurch begrenzt, dass der Stabilisator aus dem Kunststoff herausmigriert bzw. herausgelöst wird. Es hat nicht an Versuchen gefehlt, die Extrahierbarkeit der Additive zu verringern. So wurde z.B. vorgeschlagen, dem Kunststoffpulver Phenoldiazoverbindungen zuzumischen, welche bei der Verarbeitung unter Stickstoffabspaltung in Carbene zerfallen, die sich an die Kohlenwasserstoffkette des Kunststoffes anlagern [«Polymer Letters Edition», Bd. 11, S. 357-361 (1973)]. Dieses Verfahren hat den Nachteil, dass der entstehende Stickstoff zur Blasenbildung im Kunststoff führt. Derselbe Einwand ist gegen Sulfonylazid-Antioxidantien sowie gegen Azidoformyl-Antioxidantien (DE-OS Nr. 2733657 bzw. DE-OS Nr. 2636136) zu erheben.

Eine weitere Klasse von phenolischen Antioxidantien stellen Polymerisate von sterisch gehinderten Phenolen, welche in Nachbarschaft zum aromatischen Kern Vinylgruppen besitzen, dar (z.B. US-PS Nr. 4026342 sowie Japanische PS Nr. 77050-048). Der schwerwiegende Nachteil dieser Verbindungsklasse ist, dass die Polymeren bei der starken thermischen Belastung während der Verarbeitung des Kunststoffes zur Rückspaltung in die flüchtigen monomeren Ausgangsprodukte neigen.

Auch o,o'-verknüpfte polymere phenolische Antioxidantien, welche Phenylsulfonsäuregruppen im Polymerkörper enthalten, sind schon vorgeschlagen worden (DE-OS Nr. 2716811). Solche Antioxidantien mögen bei der Kabelummantelung zur Cu-Komplexierung eine gewisse Bedeutung haben, zur Stabilisierung von Polyolefinen sind sie im allgemeinen sicher nicht geeignet, da die Einwirkung von Phenylsulfonsäure auf Phenole bei den hohen Temperaturen der Polyolefinverarbeitung zu Verfärbungserscheinungen führt.

Polymere Phenolester, bei welchen die Verknüpfung der Monomereinheiten durch Veresterung der phenolischen OH-Gruppe mit mehrbasischen Säuren zustande kommt, sind ebenfalls bereits als Antioxidantien bekannt (DE-OS Nr. 2715589). Der Nachteil dieser Verbindungsklasse ist, dass durch die Esterbildung die antioxidativ wirksame Phenolgruppierung zum Teil blockiert und somit an der vollen Entfaltung ihrer Wirksamkeit gehindert wird.

Es wurden schliesslich auch polymere phenolische Antioxidantien beschrieben, welche durch Ziegler-Polymerisation von ungesättigten Alkoholen und anschliessende Umesterung mit Phenolalkancarbonsäureestern dargestellt werden (DE-AS Nr. 2119701). Dieser Syntheseweg ist aufwendig und umständlich.

Während die eingangs erwähnten niedermolekularen Carben-Vorstufen zwar homogen im Kunststoff verteilt werden können, aber die geschilderten Nachteile aufweisen, sind die angeführten polymeren Antioxidantien wegen ihres hohen Molekulargewichtes im Kunststoff in niedriger Konzentration nicht mehr homogen dispergierbar. Dies bedingt, dass man entweder hohe Stabilisatorkonzentrationen einsetzen muss, was unwirtschaftlich ist, oder dass man sich mit einer im Vergleich zu dem Stand der Technik weniger befriedigenden Stabilisierung zufrieden gibt, wenn man Wert auf eine hohe Migrationsfestigkeit des eingesetzten Stabilisators legt.

Es besteht daher ein Bedarf nach Stabilisatoren, deren Molekulargewicht einerseits zur homogenen Verteilung im Kunststoff hinreichend klein ist, die aber andererseits zum Teil noch reaktive Gruppen enthalten, welche unter den Bedingungen der Kunststoffverarbeitung zu einer Molekulargewichtserhöhung und somit zu einer Verbesserung der Migrationsfestigkeit beitragen.

Es wurde gefunden, dass bestimmte Phenolgruppen enthaltende Epoxidharze überraschenderweise die gestellten Anforderungen erfüllen.

Die vorliegende Erfindung betrifft daher Phenolgruppen enthaltende Epoxidharze und ihre Homooligomerisate der allgemeinen Formel (I)

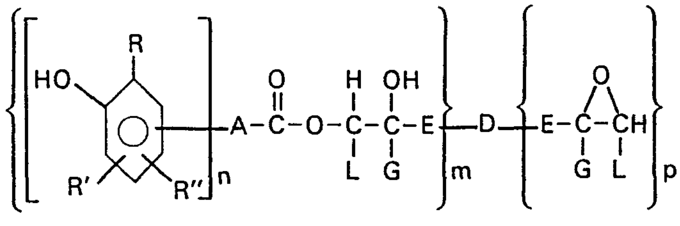

(I)

in der

R = $C_2$ bis $C_4$ Alkyl, vorzugsweise t-Butyl,

R' und R'' unabhängig voneinander H oder $C_1$ bis $C_4$ Alkyl bedeuten,

n = 1 oder 2 sein soll, und

A = eine chemische Bindung oder

α) der Rest eines geradkettigen oder verzweigten, unsubstituierten oder phenylsubstituierten Alkans mit 1 bis 20 C-Atomen, oder

β) der Rest eines unsubstituierten oder $C_1$ bis $C_6$ alkylsubstituierten, cycloaliphatischen Alkans mit 5 bis 12 C-Atomen, oder

γ) ein unsubstituierter oder $C_1$ bis $C_{12}$ alkylsubstituierter Phenyl- oder Naphthylrest ist,

E für eine $CH_2$-Gruppe,
G für ein H-Atom oder eine $CH_3$-Gruppe und
L für ein H-Atom steht, oder
E und L zusammen mit den sie verbindenden C-Atomen einen Cycloalkylrest mit 5 bis 12 C-Atomen, der auch alkylsubstituiert sein kann, bilden und
D = Sauerstoff oder ein Radikal der Formel

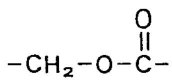

oder 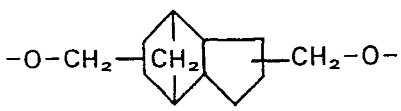

oder $-O-(CH_2)_q-O-\!\!\left[CH_2-CHOH-CH_2-O-(CH_2)_q-O-\right]_r$

oder 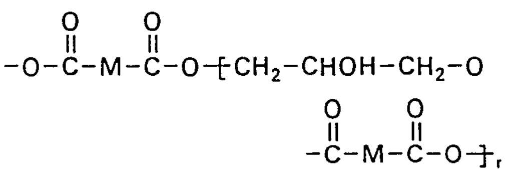

mit M = $-(CH_2)_{q-2}$, 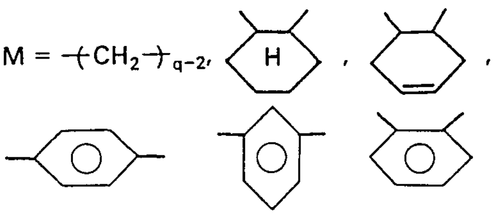

oder 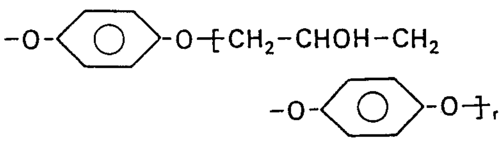

oder 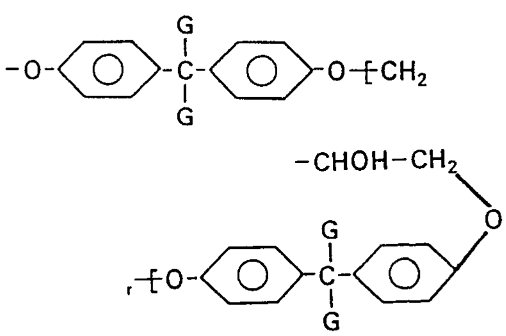

mit q = 2 bis 10 und r = 0 bis 10

oder 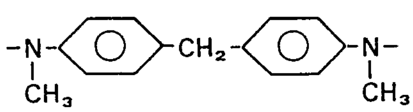

oder 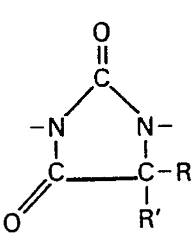

oder 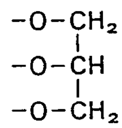 oder 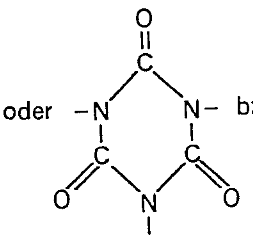 bzw. 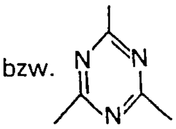

oder 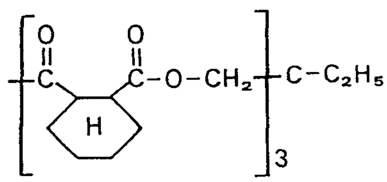

sein soll,

m = 1,2 oder 3 und
p = 0, 1 oder 2 bedeutet, mit der Einschränkung, dass p+m entweder 2 oder 3 ist.

Die Erfindung betrifft weiterhin die Verwendung der neuen Verbindungen als Stabilisatoren in synthetischen Polymeren.

Die in der allgemeinen Formel (I) dargestellte phenolische Gruppierung entstammt einer Phenolcarbonsäure der Struktur

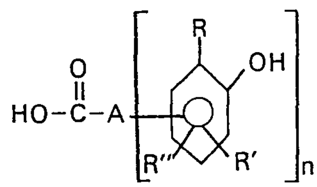

wobei A, R, R', R'' und n die oben angegebene Bedeutung besitzen. Solche Phenolcarbonsäuren mit n = 1 sind beispielsweise 3,5-Ditert.-butyl-4-hydroxyphenylpropionsäure, 3,5-Ditert.-butyl-4-hydroxybenzoesäure, 3,5-Ditert.-butyl-4-hydroxyphenylessigsäure und auch 2,4-Ditert.-butyl-3-hydroxyphenyl-6-methylbenzoesäure und ihre Abwandlungsprodukte, die z.B. in der DE-OS Nr. 2445306 näher beschrieben werden.

Phenolcarbonsäuren mit n = 2 sind Bishydroxyphenylcarbonsäuren, die beispielsweise aus der DE-OS Nr. 2544014 bekannt sind. Geeignet sind z.B. 3,3-Bis-(3'-tert.-butyl-4'-hydroxyphenyl) butansäure, Bis-(3-tert.-butyl-4-hydroxyphenyl) essigsäure, 2,2-Bis-(3'-tert.-butyl-4'-hydroxyphenyl)cyclohexancarbonsäure-(1) oder auch 2,2-Bis-(3'-tert.-butyl-4'-hydroxyphenyl) cyclohexyl-1-propionsäure. Von dieser zu bevorzugenden Verbindungsklasse seien insbesondere Bis-(3-tert.-butyl-4-hydroxyphenyl)essigsäure, 3,3-Bis-(3'-tert.-butyl-4'-hydroxyphenyl)-butansäure und 4,4-Bis-(3'-tert.-butyl-4'-hydroxyphenyl)pentansäure genannt.

Die erfindungsgemässen Substanzen werden durch Umsetzen von Phenolcarbonsäuren mit Epoxidverbindungen erhalten. So addiert man z.B. 1 oder 2 mol Phenolcarbonsäure an Diglycidylether, Bis(glycidyloxymethyl)tricyclododecan, Alkylendioldiglycidylether, an α,ω-Bisglycidylalkylendiolglycerinpolyether, an Diglycidylester, an Di-β-methylglycidylester von ggf. alkyl-

verzweigten aliphatischen, cycloaliphatischen oder aromatischen Dicarbonsäuren, an α,ω-Bisglycidyldicarbonsäureglycerinpolyester, an Resorcin- bzw. Hydrochinondiglycidylether, an α,ω-Bisglycidylhydrochinonglycerinpolyether, an Bisphenol-F- bzw. Bisphenol-A-diglycidylether, an deren α,ω-Diglycidylglycerinpolyether, an N,N'-Bisglycidyl-N,N'-dimethylaminodiphenylmethan oder an N,N'-Bisglycidylhydantoine.

Man kann auch 1, 2 oder 3 mol Phenolcarbonsäure mit einem mol Glycerintriglycidylether oder Triglycidylisocyanurat oder 2,4,6-Trisepoxypropyltriazin oder mit Trimethylolpropantris-(2,3-epoxypropylhexahydrophthalat) zur Reaktion bringen, oder 1 oder 2 mol Phenolcarbonsäure an beispielsweise Bis-(2,3-epoxycyclopentyl)ether oder 3,4-Epoxycyclohexylmethyl-3,4-epoxy cyclohexancarboxylat oder Bis-(3,4-epoxy-6-methylcyclohexylmethyl)adipat oder auch an Bis-(3,4-epoxycyclohexylmethyl)adipat addieren.

Bei der Umsetzung werden in der Regel die freien Phenolcarbonsäuren mit den Epoxidverbindungen bei 50 bis etwa 200, vorzugsweise bei 80 bis 130° C zur Reaktion gebracht. Sie kann sowohl ohne Lösungsmittel durchgeführt werden, als auch in Gegenwart von inerten aromatischen oder aliphatischen Lösungsmitteln wie Xylol, Toluol oder Diisobutylketon, analog einem in der DE-OS Nr. 2449847 beschriebenen Verfahren. Arbeitet man lösungsmittelfrei, so kann die Reaktion in Mischern, Knetern oder auf Walzen durchgeführt werden.

Bevorzugt wird die Umsetzung von Phenolcarbonsäuren mit Di- und Polyepoxidverbindungen des Handels, deren Darstellung in der Literatur ausführlich beschrieben ist, beispielsweise in Houben-Weyl, 14/2 (1963), S. 462 ff., oder in «Praktikum der Makromolekularen Organischen Chemie», Hüthig-Verlag, Heidelberg, 1966, S. 218 ff.

Derartige Polyepoxidverbindungen sind z.B. Polyglycidyl- bzw. Poly-β-methylglycidylether von mehrwertigen Alkoholen wie Polyethylenglykolen, Polypropylenglykolen oder 2,2-Bis-(4'-hydroxycyclohexy)propan, von mehrwertigen Phenolen wie 2,2-Bis-(4'-hydroxyphenyl)-propan, Bis-(4-hydroxyphenyl)sulfon, 1,1,2,2-Tetrabis-(4-hydroxypehnyl)ethan, ferner Polyglycidyl- bzw. Poly-β-methylglycidyl)ester von Polycarbonsäure, z.B. Phthalsäurediglycidylester, Isophthalsäurediglycidylester, $\Delta^4$-Tetrahydrophthalsäurediglycidylester, Hexahydrophthalsäurediglycidylester, Glutarsäurediglycidylester, Adipinsäurediglycidylester, Sebazinsäurediglycidylester, alkylsubstituierte Dicarbonsäuredi-(β-methylglycidyl)ester, wie sie z.B. in der DE-AS Nr. 1942836 beschrieben sind, Triglycidylisocyanurat, N,N'-Diglycidyl-5,5-dimethylhydantoin oder auch Epoxidverbindungen, die aus der Dehydrohalogenierung der Reaktionsprodukte aus Epihalogenhydrin bzw. β-Methylepihalogenhydrin und primären oder sekundären Aminen wie Anilin oder 4,4'-Diaminodiphenylmethan erhalten werden; ferner, mehrere Epoxidgruppen enthaltende alicyclische Verbindungen wie Vinylcyclohexendiepoxid, Dicyclopentadiendiepoxid, Äthylenglykolbis-(3,4-epoxytetrahydrodicyclopentadien-8-yl)ether, (3',4'-Epoxycyclohexylmethyl)-3,4-epoxycyclohexancarboxylat, (3',4'-Epoxy-6'-methylcyclohexylmethyl)-3,4-epoxy-6-methylcyclohexancarboxylat, Bis-(2,3-epoxycyclopentyl)ether, 3-(3',4'-Epoxycyclohexyl)-2,4-dioxaspiro-(5,5)-9,10-epoxyundecan, 3,4-Epoxyhexahydrobenzol-3', 4'-epoxy-1',1'-bis(oxymethyl)cyclohexan, Bis-(3,4-epoxy-6-methylcyclohexyl)adipat oder Bis-(3,4-epoxycyclohexylmethyl)adipat.

Gewünschtenfalls kann man eine oder auch mehrere der aufgeführten Polyepoxidverbindungen einsetzen, gegebenenfalls auch im Gemisch mit sogenannten reaktiven Verdünnern wie z.B. Styroloxid, Butylglycidylether, Isooctylglycidylether, Phenylglycidylether, Kresylglycidether oder Glycidylestern von synthetischen hochverzweigten, in der Hauptsache tertiären aliphatischen Monocarbonsäuren.

Es ist ferner möglich, die erhaltenen, noch Epoxidgruppen enthaltenden Additionsprodukte aus Polyepoxid und Phenolcarbonsäure nachträglich in ihrem Molekulargewicht zu steigern. Dies wird erreicht durch Reagierenlassen mit sog. Härtern. Als solche kommen basische oder saure Verbindungen in Frage, beispielsweise Amine oder Amide wie aliphatische, cycloaliphatische oder aromatische primäre, sekundäre und tertiäre Amine wie Ethanolamin, Ethylendiamin, Hexamethylendiamin, Trimethylhexamethylendiamin, Diethylentriamin, Triethylentetramin, Tetraethylenpentamin, 2,2,6,6-Tetramethyl-4-aminopiperidin, 2,2,6,6-Tetramethyl-4-hydroxypiperidin, N,N-Dimethylpropylendiamin-1,3, N,N-Diethylpropylendiamin-1,3, Bis-(4-amino-3-methylcyclohexyl)methan, 3,5,5-Trimethyl-3-(aminomethyl)cyclohexylamin («Isophorondiamin»), Mannichbasen wie 2,4,6-Tris(dimethylaminomethyl)phenol, m-Phenylendiamin, p-Phenylendiamin, Bis-(4-aminophenyl)methan, Bis-(4-aminophenyl)sulfon, m-Xylylendiamin, N-(2-aminoethyl)piperazin.

Als saure Härter sind u.a. mehrbasische Carbonsäuren und ihre Anhydride zu nennen, z.B. Phthalsäureanhydrid, $\Delta^4$-Tetrahydrophthalsäureanhydrid, Hexahydrohthalsäureanhydrid, 4-Methylhexahydrophthalsäureanhydrid, 3,6-Endomethylen-$\Delta^4$-tetrahydrophthalsäureanhydrid, 4-Methyl-3,6-endomethylen-$\Delta^4$-tetrahydrophthalsäureanhydrid, 4-Methyl-3,6-endomethylen-$\Delta^4$-tetrahydrohthalsäureanhydrid (= Methylnadicanhydrid), 3,4,5,6,7,7-Hexachlor-3,6-endomethylen-$\Delta^4$-tetrahydrophthalsäureanhydrid, Bernsteinsäureanhydrid, Adipinsäureanhydrid, Azelainsäureanhydrid, Sebacinsäureanhydrid, Maleinsäureanhydrid, Dodecylbernsteinsäureanhydrid, Pyromellitsäureanhydrid oder Gemische solcher Anhydride.

Die Umsetzung mit den genannten Polycarbonsäureanhydriden lässt sich durch Zusatz basischer Verbindungen wie Alkalialkoholat, tert.-Amine, deren Salze oder quarternäre Amoniumverbindungen beschleunigen. Genannt seien z.B. Benzyldimethylanilin, 4-Aminopyridin, 4-(Dimethylamino)pyridin und insbesondere Alkalimethylat, -ethylat oder Isopropylat. Auch saure Verbindungen wie z.B. phosphorige- oder Phosphorsäure sind geeignet. Die Katalysatormengen liegen bei 0,001 bis 1 Gew.%, bezogen auf Epoxid.

Ein nicht zu unterschätzender Vorteil der erfindungsgemässen Substanzklasse ist, dass die als Ausgangsprodukte dienenden Epoxidverbindungen grosstechnisch hergestellt werden, und auf dem Lacksektor, beispielsweise zur Lackierung von Konservendosen, verbreitet Anwendung finden. Im Verein mit den aus billigen Ausgangsstoffen in einfacher Weise gut zugänglichen Phenolcarbonsäuren und dem glatt verlaufenden erfindungsgemässen Herstellungsverfahren können die polymeren Stabilisatoren gemäss der Erfindung somit preisgünstig dargestellt werden.

Neben der einfachen Darstellbarkeit aus gut zugänglichen Ausgangsprodukten ist bei den erfindungsgemässen Stabilisatoren noch die hohe Verfärbungsstabilität beim Einsatz in den Kunststoffen hervorzuheben. Dieses ist überraschend und war nicht vorhersehbar, insbesondere bei den Phenolcarbonsäureestern der Glycerinabkömmlinge. Wie aus der Chemie der Fette bekannt ist, neigen Glycerincarbonsäureester bei thermischer Belastung zur Bildung von verfärbten Zersetzungsprodukten. Um so erstaunlicher ist, dass die ähnlich gebauten erfindungsgemässen Verbindungen sogar noch ein besseres Farbverhalten als handelsübliche phenolische Antioxidantien aufweisen.

Weiterhin ist die geringe Flüchtigkeit der erfindungsgemässen Stabilisatoren hervorzuheben. Während nämlich die eingangs erwähnten Polymerisate ethylenisch ungesättigter Verbindungen unter der starken thermischen Belastung bei der Kunststoffverarbeitung — wie bereits erwähnt — die Tendenz haben, in Monomeren zurückzuspalten, neigen die erfindungsgemässen Verbindungen unter diesen Bedingungen sogar noch zu einer Molekulargewichtserhöhung, da die im Oligomerisat noch vorhandenen Epoxidgruppen mit sich selbst aber gegebenenfalls auch mit zugesetzten Aminen, Carbonsäuren oder auch Carbonsäureanhydriden zu höhermolekularen Substanzen reagieren können. Dieses Verhalten ist deshalb besonders vorteilhaft, weil sich auf diese Weise präpolymere Antioxidantien mit einem zur homogenen Verteilung im Kunststoff noch optimalen, vergleichsweise niederen Molekulargewicht bei der Verarbeitung im Kunststoff homogen einarbeiten und verteilen lassen, und sie gleichzeitig unter dem Einfluss der hohen Verarbeitungstemperaturen zu höhermolekularen, migrationsfesten Stabilisatoren weiterreagieren können.

Dieses günstige Verhalten der erfindungsgemässen Substanzen ist bei keinem der bisher beschriebenen Stabilisatorsysteme anzutreffen und stellt einen erheblichen Fortschritt gegenüber dem Stand der Technik dar.

Die erfindungsgemässen Phenolgruppen enthaltenden Epoxidharze stellen vorzugsweise Präpolymere mit einem nicht allzu hohen Molekulargewicht, etwa in der Grössenordnung 1000 bis 10 000, insbesondere 2000 bis 5000 dar. Man erhält sie durch Addition von 0,01 bis maximal 1 mol Phenolcarbonsäure an ein Epoxidäquivalent einer Polyepoxidverbindung, wobei die Reaktionsbedingungen so zu wählen sind, dass die Ausbildung vernetzter, hochpolymerer Reaktionsprodukte vermieden wird. Man erreicht dies beispielsweise durch Verdünnen der Reaktionskomponenten mit einem Lösungsmittel, durch eine niedrige Reaktionstemperatur von etwa 50 bis 110°C und vor allem durch rechtzeitigen Abbruch der Reaktion durch Temperaturabsenkung, sobald sich — etwa durch Trübung des Reaktionsansatzes — die Abscheidung von unlöslichen, vernetzten Hochpolymeren anzeigt.

Die erfindungsgemässen Stabilisatoren zeichen sich durch ihre hohe Extraktionsfestigkeit aus. Eine hohe Extraktionsfestigkeit des Stabilisators im Kunststoff auch gegenüber öl- und fettähnlichen Substanzen ist deshalb von entscheidender Bedeutung, weil einerseits der beabsichtigte Oxidationschutz des Kunststoffs auch in Kontakt mit Ölen und Fetten erhalten bleibt und vor allem, weil darüber hinaus die äusserst unerwünschte Kontaminierung von gegebenenfalls in dem Kunststoff verpackten fett- und ölhaltigen Lebensmitteln mit den Stabilisatoren unterbleibt.

Die Phenolgruppen enthaltenden Epoxidharze der Erfindung sind hervorragend zur Stabilisierung von synthetischen Polymerisaten und Copolymerisaten von $C_2$- bis $C_4$-α-Olefinen, vorzugsweise von Polyolefinen wie Polybutadien, Polyisopren und insbesondere Polyethylen und Polypropylen geeignet, ferner für Polystyrol, Polyacrylate und Polymethacrylate, auch bei den durch Glasfasern, Asbest usw. verstärkten Typen. Sie kommen in Mengen von 0,001 bis 5,0, vorzugsweise 0,01 bis 1,0 Gewichtsteilen auf 100 Gewichtsteile Polymerisat, zum Einsatz. Bevorzugtes Anwendungsgebiet finden sie vor allem dort, wo der Kunststoff eluierend wirkenden Medien wie Öl, Fett, organischen Lösemitteln aber auch Wasser, Säuren und Laugen ausgesetzt ist.

Beim Stabilisieren der Polymeren werden als Costabilisatoren gegebenenfalls noch übliche andere phenolische Antioxidantien zugesetzt. Aus der grossen Zahl der hierfür in Frage kommenden Produkte seien beispielsweise genannt: Ester der 3,5-Ditert.-butyl-4-hydroxyphenylpropionsäure oder der 3,3-Bis-(3'-tert.-butyl-4'-hydroxyphenyl)buttersäure. Als pH-Stabilisator finden Erdalkalisalze von langkettigen Carbonsäuren Verwendung, z.B. Ca-Stearat oder Ca-Montanat. In manchen Fällen erweist sich der Zusatz von Schwefel-Costabilisatoren vorteilhaft, z.B. von Lauryl- oder Stearylthiodipropionat oder Dioctadecylsulfid bzw. Dioctadecyldisulfid als vorteilhaft. Als phosphorhaltige Costabilisatoren sind beispielsweise Phosphite, Phosphinate und Phosphonate zu nennen, wie etwa Distearylpentaerythrityldiphosphit, Distearyl-β-hydroxytriacontylsorbityltriphosphit, Tris-(2,4-ditertbutylphenyl)-phosphit, Diphenyl-4,4'-diphosphinsäuretetrakis-(2,4-ditert.-butylphenyl)ester oder das Ca-

bzw. Ni-Salz des 3,5-Ditert.-butyl-4-hydroxybenzylphosphonsäureethylesters. Gelegentlich empfiehlt es sich, neben den genannten Costabilisatoren noch Lichtschutzmittel einzusetzen, von denen als Beispiel Oxybenzophenone, Benztriazole oder auch die Piperidinstabilisatoren, insbesondere die hochpolymeren Vertreter dieser Substanzklasse, genannt seien.

Die nachfolgenden Beispiele sollen die Erfindung näher erläutern.

*Beispiel 1*

Oligomere des Glycerinbis-(2-hydroxy-3-[3',3'-di-(3''-tert.-butyl-4''-hydroxyphenyl)-butyroxy]propyl)monoglycidyltriethers

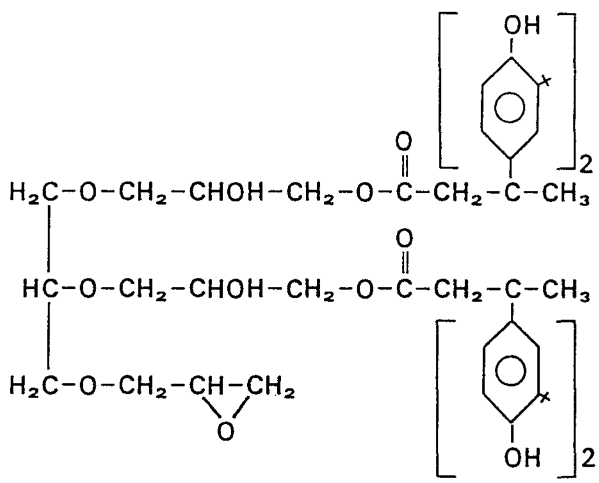

10 g (1/30 mol) Trisglycidylglycerinether (erhalten nach «Praktikum der Makromolekularen Organischen Chemie», Hüthig-Verlag, Heidelberg, 1966, S. 222), 25,3 g (2/30 mol) 3,3-Bis-(3'-tert.-butyl-4'-hydroxyphenyl)butansäure (hergestellt nach DE-OS Nr. 2544014) und 0,05 g KOH werden in 50 ml Kylol 4 h unter Rückfluss gekocht. Im Dünnschichtchromatogramm ist nach dieser Zeit keine 3,3-Bis-(3'-tert.-butyl-4'-hydroxyphenyl)butansäure mehr nachweisbar. Nun wird das Lösungsmittel im Wasserstrahlvakuum abdestilliert. Der verbleibende Rückstand, ein nahezu farbloses Glas vom Fp/Tp 100/109° C, besitzt ein dampfdruckosmometrisch bestimmtes Molekulargewicht von 1230 (MG der Th. 1061), was einem Polymerisationsgrad von 1,16 entspricht.

a) 5 g des erhaltenen Produktes werden bei 200° C 2 h getempert. Das Molekulargewicht erhöht sich durch diese Massnahme auf 2800, der Fp/Tp auf 115/120° C.

b) 5 g des erhaltenen Produktes werden mit 0,5 g Tetrahydrophthalsäureanhydrid vermischt, aufgeschmolzen und 1 h bei 150° C getempert. Man erhält ein Produkt mit der Molmasse 3140, was einem Polymerisationsgrad von 2,6 entspricht. Der Fp/Tp hat sich auf 119/130° C erhöht.

c) 5 g des Produktes werden mit 0,5 g Terephthalsäure 2 h, wie vorstehend angegeben, bei 200° C getempert. Das Produkt ist in allen Lösemitteln unlöslich und unschmelzbar.

*Beispiel 2*

Oligomere des Glycerinmono-(2-hydroxy-3-[3',3'-di-(3''-tert.-butyl-4''-hydroxyphenyl)-butyroxy]propyl)bisglycidyltriethers

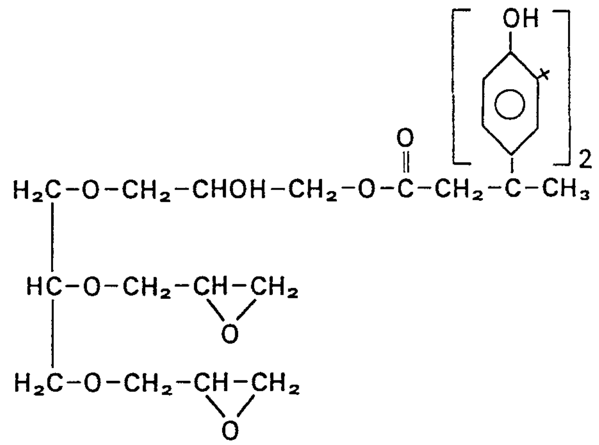

10 g (1/30 mol) Trisglycidylglycerinether, 12,7 g (1/30 mol) 3,3-Bis-(3'-tert.-butyl-4'-hydroxyphenyl)butansäure und 0,05 g KOH werden in 50 ml Xylol 90 min unter Rückfluss gekocht.

Im Dünnschichtchromatogramm ist dann keine Phenolcarbonsäure mehr nachweisbar. Der Reaktionsansatz wird mit 0,5 ml Eisessig neutralisiert und das Lösungsmittel abdestilliert. Man erhält ein leicht gelblich verfärbtes, glasiges Harz, dessen Molekulargewicht bei 2240 liegt. 15- bis 30minutiges Tempern bei 200° C/Vakuum erhöht das Molekulargewicht auf 2410 bzw. 2510, was einem Polymerisationsgrad von etwa 3 bis 4 entspricht.

*Beispiel 3*

Oligomere des Glycerinbis-(2-hydroxy-3-[4',4'-di-(3''-tert.-butyl-4''-hydroxyphenyl)pentanoxyloxy]propyl)glycidylethers

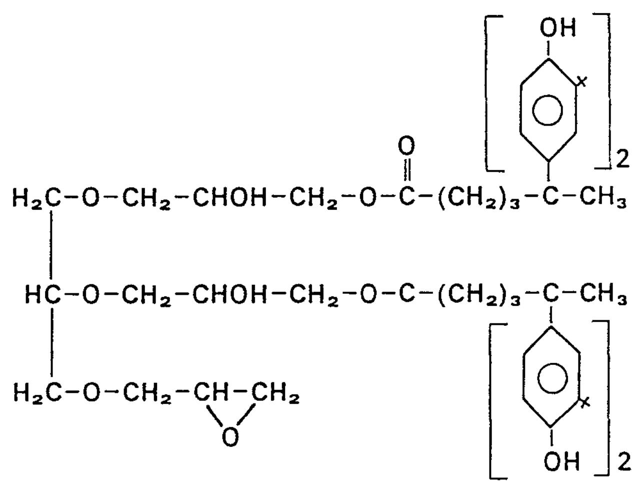

Analog zu Beispiel 1 werden 5 g (1/60 mol) Trisglycidylglycerinether, 13,3 g (2/60 mol) 4,4-Bis-(3'-tert.-butyl-4'-hydroxyphenyl)pentansäure (hergestellt aus Lävulinsäure und o-tert.-butylphenol analog einer in DE-OS Nr. 2544014 beschriebenen Methode) in 50 ml Xylol in Gegenwart von 0,05 g KOH 5 h unter Rückfluss gekocht. Laut Dünnschichtchromatogramm ist nach dieser Zeit die Phenolcarbonsäure abreagiert. Nach dem Abziehen des Lösemittels verbleibt als Rückstand ein farbloses, glasartiges Harz mit einem Molekulargewicht von 1830, was einem Polymerisationsgrad von ca. 1,7 entspricht.

*Beispiel 4*

Oligomere von Terephthalsäuremonoglycidyl-mono-(2-hydroxy-3-[3',3'-di-(3"-tert.-butyl-4"-hydroxyphenyl)butyroxy]propylester

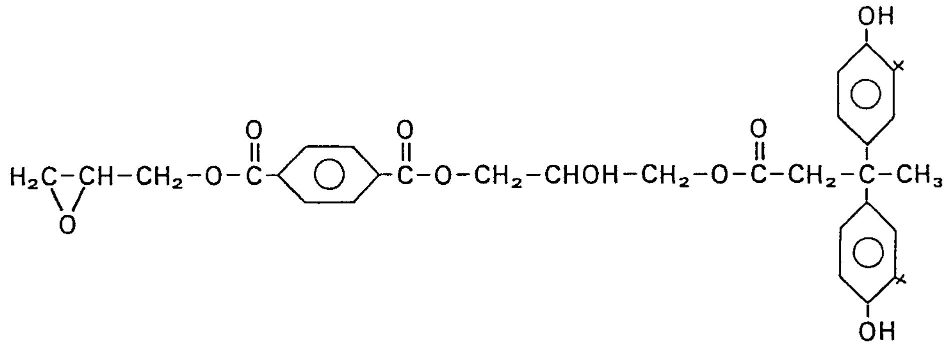

Analog Beispiel 1 werden 27,8 g (0,1 mol) Terephthalsäurediglycidylester und 38,4 g (0,1 mol) 3,3-Bis-(3'-tert.-butyl-4'-hydroxyphenyl)butansäure in 250 ml Xylol in Gegenwart einer Spur KOH 3 h unter Rückfluss gekocht. Man erhält nach dem Abdampfen des Lösungsmittels 60 g eines spröden Glases mit dem Fp/Tp 116/123° C und einem MG von 955, was einem Polymerisationsgrad von 1,4 entspricht.

Tempert man 7,3 g dieses Produktes mit 0,55 g Tetrahydrophthalsäureanhydrid 1 h bei 150° C, so erhält man einen Stoff mit dem Molgewicht 1540, was einem Polymerisationsgrad von 2,3 entspricht. Der Fp/Tp liegt bei 119/136° C.

*Beispiel 5*

Terephthalsäure-(2-hydroxy-3-[3',3'-di-(3"-tert.-butyl-4"-hydroxyphenyl)butyroxy]propyldiester

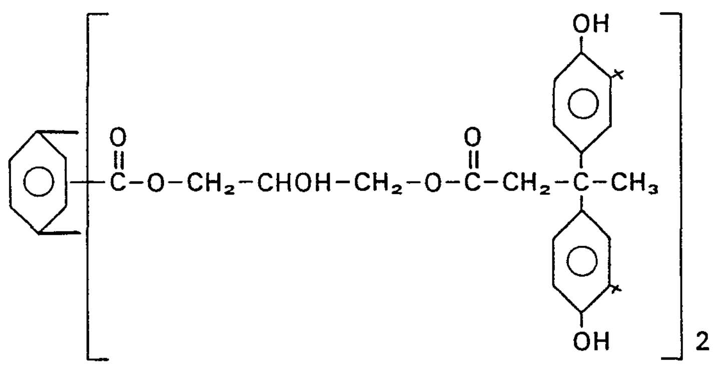

Analog zu Beispiel 1 werden 15,3 g (0,055 mol) Terephthalsäurediglycidylester und 38,4 g (0,10 mol) 3,3-Bis-(3'-tert.-butyl-4'-hydroxyphenyl)butansäure in 250 ml Xylol in Gegenwart einer Spur NaOH 24 h am Rückfluss gekocht. Nach Zugabe von weiteren 1,5 g Terephthalsäurediglycidylester wird nochmals 4 h gekocht. Das nach dem Neutralisieren mit etwas Eisessig nach dem Abdampfen des Lösemittels als Rückstand verbleibende Produkt hat einen Fp/Tp von 112/123° C und ein Molgewicht von 1160 (Th. 1074).

*Beispiel 6*

Oligomere von Bisphenol-A/Bisphenol-F-monoglycidylmono-(2-hydroxy-3-[3',3'-di-(3"-tert.-butyl-4"-hydroxyphenyl)butyroxy]propylether

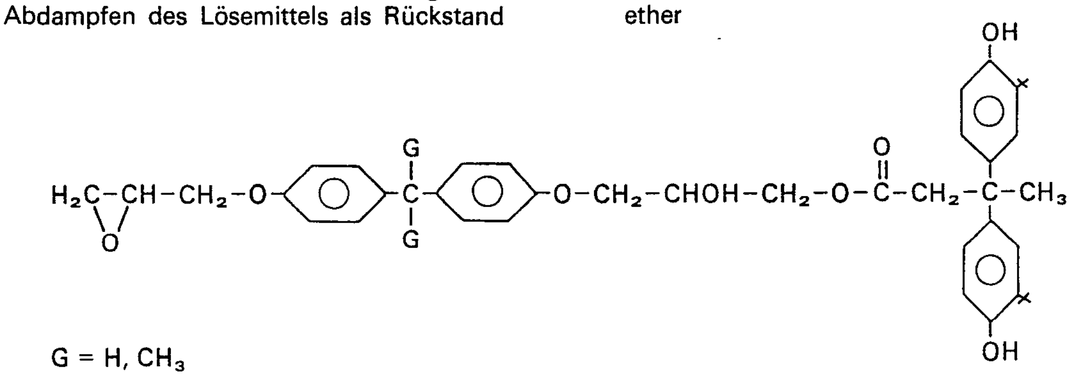

G = H, $CH_3$

18,3 g (0,05 mol) eines handelsüblichen Gemisches aus ca. 70% Bisphenol-A-diglycidylether und 30% Bisphenol-F-diglycidylether [2,2-(4,4'-Dihydroxydiphenyl)dimethylmethan bzw. -methan] in 200 ml Dimethylformamid werden im Laufe von 2 h zu einer auf 130°C erwärmten Lösung von 19,2 g (0,05 mol) 3,3-Bis-(3'-tert.-butyl-4'-hydroxyphenyl)butansäure und 0,05 g KOH in 50 ml Dimethylformamid getropft. Man rührt 4 h nach, versetzt dann mit weiteren 2 g Bisphenol-A/F-diglycidylether und destilliert nach 2 h das Lösungsmittel im Vakuum ab.

Man erhält ein Harz mit dem Fp/Tp von 107/118°C. Die Bestimmung der Molmasse ergibt einen Wert von 1965, was einem Oligomerisationsgrad von 2,6 entspricht.

*Beispiel 7*

Oligomere von

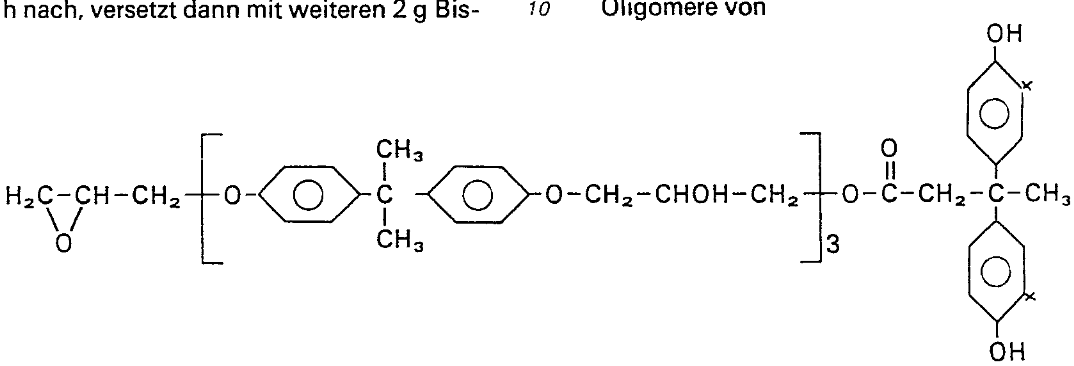

73,3 g (0,15 Epoxyäquivalent) eines käuflichen α,ω-Diglycidylethers von trimerem Bisphenol-A-(β-hydroxypropandiol)ethers in 200 ml Dimethylformamid werden unter $N_2$-Überlagerung im Laufe von 2 h zu einer auf 140°C erwärmten Lösung von 19,2 g (0,05 mol) 3,3-Bis-(3'-tert.-butyl-4'-hydroxyphenyl)butansäure und einer Spur KOH in 50 ml Dimethylformamid getropft.

Dann wird 6 h bei 150°C nachgerührt. Nach dem Abdestillieren des Lösungsmittels verbleibt ein leicht gelblich gefärbtes Harz mit einem Fp/Tp 148/153°C. Die Bestimmung der Molekulargewichts ergab einen Wert von 5505, was einem Oligomerisationsgrad von etwa 5 entspricht.

*Beispiele 8 bis 15*

Analog der in Beispiel 1 angegebenen Vorschrift wurden weitere Polyepoxide mit der 3,3-Bis-(3'-tert.-butyl-4'-hydroxyphenyl)butansäure (BBB) im angegebenen Molverhältnis miteinander umgesetzt.

| Beispiel Nr. | Polyepoxid | Mol BBB pro Mol Polyepoxid | Fp/Tp des Umsetzungs-produktes in °C | Molgewicht |
|---|---|---|---|---|
| 8 | 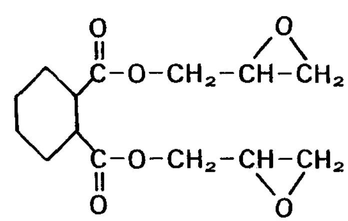 | 1 | 144/149 | 6400 |
| 9 | 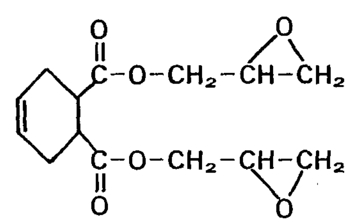 | 1 | 178/184 | — |
| 10 | 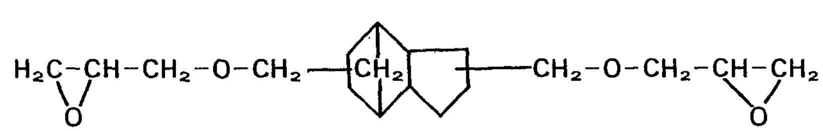 | 1 | 78/82 | — |
| 11 | 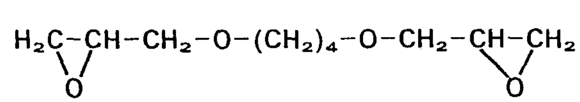 | 1 | 94/106 | 2160 |

| Beispiel Nr. | Polyepoxid | Mol BBB pro Mol Polyepoxid | Fp/Tp des Umsetzungs-produktes in °C | Molgewicht |
|---|---|---|---|---|
| 12 | 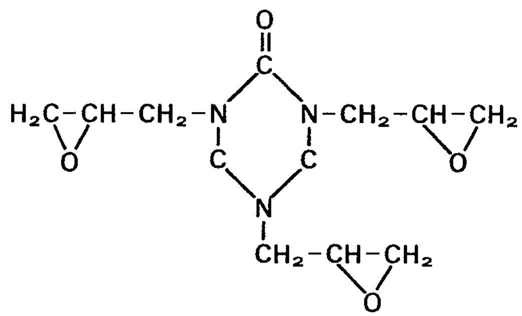 | 1,5 | 130/135 | 810 |
| 13 | 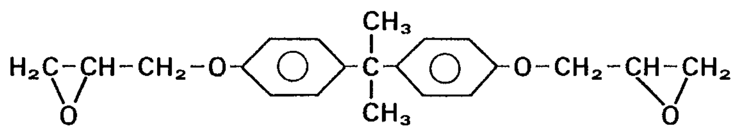 | 1 | 86/93 | 750 |
| 14 | 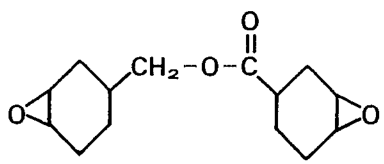 | 1 | 148/158 | 2170 |
| 15 | 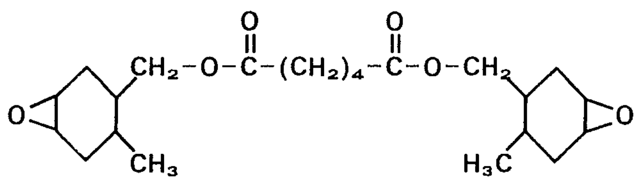 | 1 | 150/154 | 3830 |

*Beispiele 16 bis 31*

Anstelle von BBB wurden andere Phenolcarbonsäuren mit Polyepoxiden analog Beispiel 1 umgesetzt, wobei ohne Lösungsmittel gearbeitet wurde. Katalysator war eine Spur 4-Dimethylaminopyridin. Es wurde stets 1 mol Polyepoxid eingesetzt.

| Beispiel Nr. | Polyepoxid | ... Mol Phenolcarbonsäure | | Fp/Tp (°C) |
|---|---|---|---|---|
| 16 | 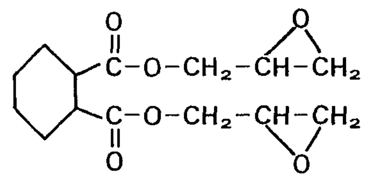 | 1 | 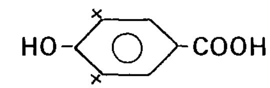 | 128/130 |
| 17 | 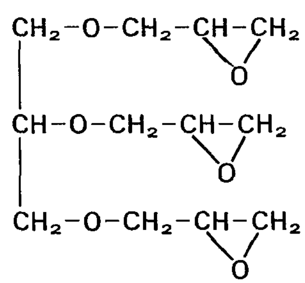 | 2 | dito | 72/76 |
| 18 | 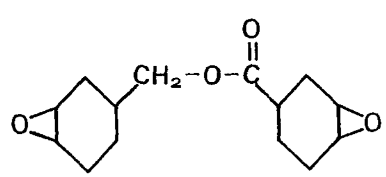 | 1 | 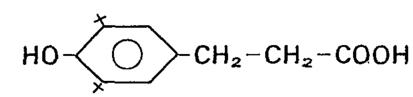 | 66/72 |
| 19 | 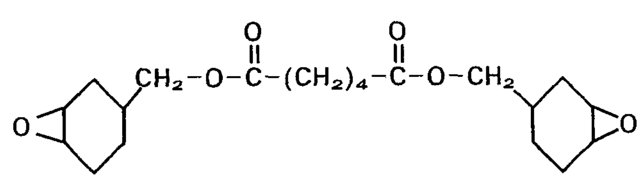 | 1 | 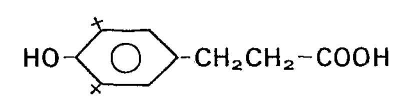 | 60/67,5 |

| Beispiel Nr. | Polyepoxid | ... Mol Phenolcarbonsäure | Fp/Tp (°C) |
|---|---|---|---|
| 20 | 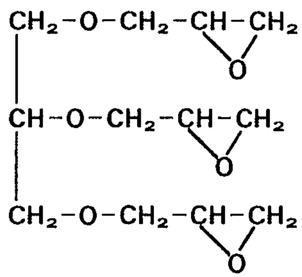 | 3 dito | 124/128 |
| 21 | 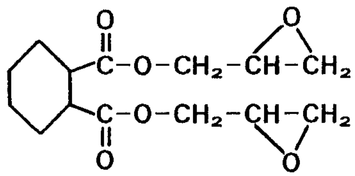 | 1 dito | 70/74 |
| 22 | 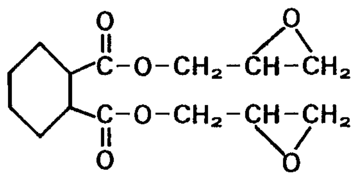 | 1 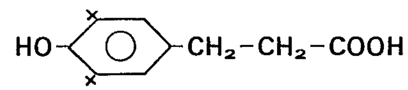 | 150 |
| 23 | 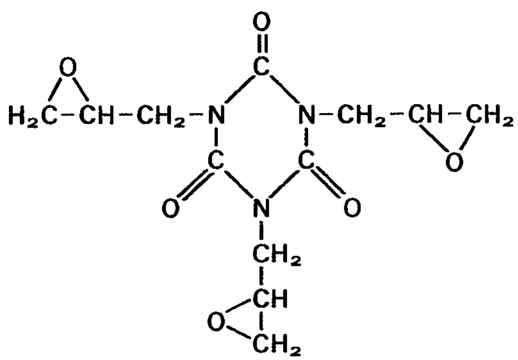 | 1,5 dito | 110/117 |

| Beispiel Nr. | Polyepoxid | ... Mol Phenolcarbonsäure | Fp/Tp (°C) |
|---|---|---|---|
| 24 | 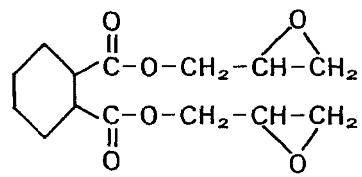 | 1 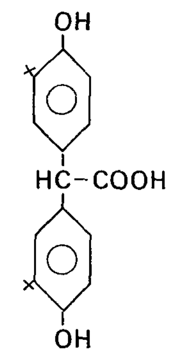 | 136/140 |
| 25 | 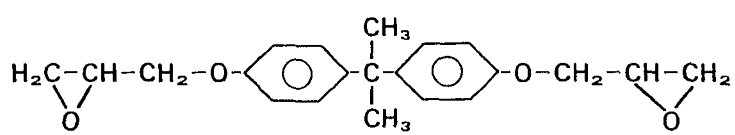 | 1 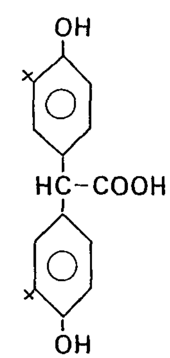 | 103/109 |
| 26 | 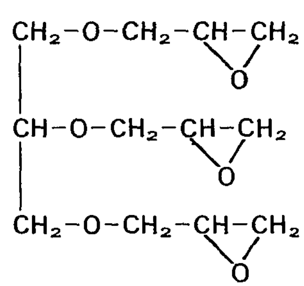 | 1 dito | 132/136 |

| Beispiel Nr. | Polyepoxid | ... Mol Phenolcarbonsäure | Fp/Tp (°C) |
|---|---|---|---|
| 27 | 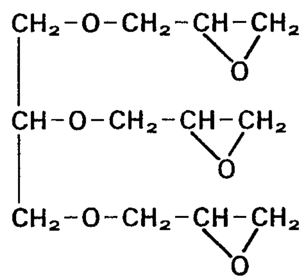 | 3 dito | 119/123 |
| 28 | 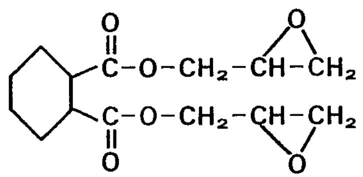 | 1 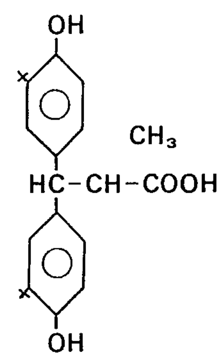 | 129/142 |
| 29 | 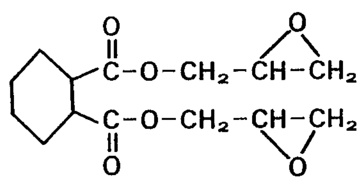 | 1 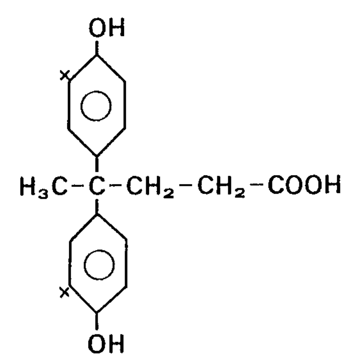 | 136/140 |

| Beispiel Nr. | Polyepoxid | ... Mol Phenolcarbonsäure | Fp/Tp (°C) |
|---|---|---|---|
| 30 | 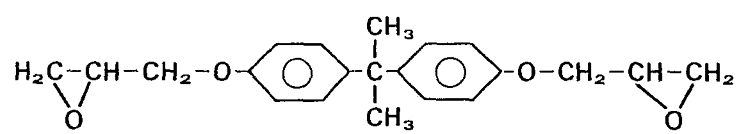 | 1 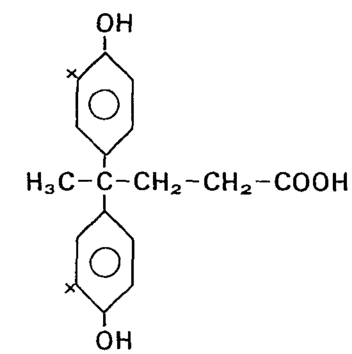 | 87/92 |
| 31 | 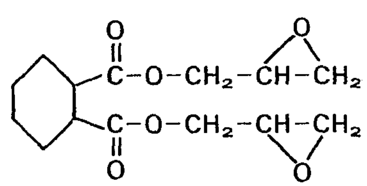 | 1 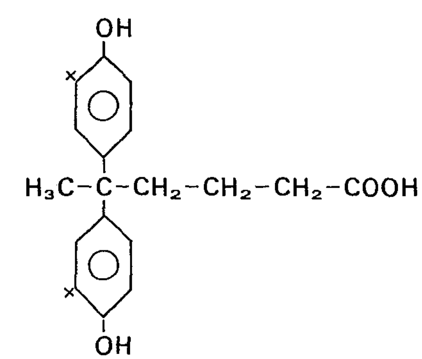 | 132/136 |

*Beispiel 32*

Dieses Beispiel soll die Schwerflüchtigkeit und Verfärbungsstabilität der erfindungsgemässen Substanzen aufzeigen.

Zur Prüfung dieser Eigenschaften werden etwa 1 g Substanz in ein Wägegläschen eingewogen und anschliessend in einem Wärmeschrank bei 200° C 2 h an der Luft stehengelassen. In nachstehender Tabelle ist der ermittelte Gewichtsverlust in Prozent angegeben.

| Probe nach Beispiel | Gewichtsverlust in % |
|---|---|
| 1 | 0,28 |
| 4 | 0,47 |
| 5 | 0,68 |
| 8 | 0,20 |
| 10 | 0,33 |
| zum Vergleich: p-Hydroxy-m,m'-ditert.-butylphenylpropionsäure-pentaerythritester | 0,74 |

Die Produkte sind durchwegs nur leicht nach gelb verfärbt, entsprechend einer Jodfarbzahl (nach DIN 6162) zwischen 5 und 12.

*Beispiel 33*

Dieses Beispiel zeigt die Wirksamkeit erfindungsgemässer Antioxidantien in einem Polypropylenwachs vom Molekulargewicht 2000 und einer Schmelzviskosität (gemessen bei 170° C) von 1550 mPas bei dessen thermischer Belastung.

Zu 100 g Wachs werden jeweils 0,5 g der erfindungsgemässen Stabilisatoren gegeben, worauf die Mischung in einem 250-ml-Dreihalskolben bei einer Innentemperatur von 150° C unter gleichzeitigem Durchleiten eines trockenen Luftstromes von 75 ml/min und Rühren mit einem Schleifenrührer mit einer Geschwindigkeit von 200 UpM 5 h gerührt wird. Dann wird der Versuch abgebrochen. Mit Hilfe eines Rotationsviskosimeters wird sodann bei 170° C die Schmelzviskosität der Proben bestimmt.

Die nachstehende Tabelle enthält die ermittelten Werte.

| Substanz nach Beispiel Nr. | Schmelzviskosität bei 170° C (in mPas) |
|---|---|
| 1 | 984 |
| 5 | 1100 |
| 11 | 776 |
| 23 | 1171 |
| zum Vergleich: | |
| ohne Stabilisator | 501 |
| handelsüblicher Stabilisator +) | 915 |

+) 3,3-Bis-(3'-tert.-butyl-4'-hydroxyphenyl)-butansäureethandioldiester

Aus der Tabelle geht hervor, dass die erfindungsgemässen Stabilisatoren den durch Wärme- und Sauerstoffeinwirkung bedingten Viskositätsabbau eindrucksvoll verzögern und in dieser Hinsicht mit handelsüblichen, niedermolekularen Phenolstabilisatoren vergleichbar sind.

*Beispiel 34*

In diesem Beispiel soll die antioxidative Wirksamkeit der erfindungsgemässen Stabilisatoren aufgezeigt werden.

Auf der Zweiwalze wird eine Mischung aus
100 Gew.-Teilen unstabilisiertem Polypropylenpulver der Dichte 0,90 (Schmelzindex $i_5$ ca. 6 g/10 min, bestimmt in Anlehnung an ASTM D 1238-62 T),

0,2 Gew.-Teilen Calciumstearat und
0,3 Gew.-Teilen Stabilisator

bei 200° C 5 min lang homogenisiert. Die Kunststoffschmelze wird sodann bei 200° C zu einer Platte von 1 mm Dicke gepresst. Aus der Kunststoffplatte werden streifenförmige Prüfkörper (100×10×1 mm) gestanzt. Diese werden zur Bestimmung der Wärmealterungsbeständigkeit in einem Umluft-Trockenschrank in ein motorgetriebenes Gestell mit rotierenden Horden eingehängt und bei einer gleichmässigen Frischluftzufuhr einer Temperaturbelastung von 140° C unterworfen. Die Zeit, nach der an einigen Stellen eine beginnende lokale Versprödung eintritt, nach DIN 53383 gekennzeichnet durch die Bildung verfärbter, trüber, teilweise abbröckelnder Stellen, wird festgehalten. Die Prüfergebnisse sind in nachstehender Tabelle zusammengefasst.

| Verbindung nach Beispiel Nr. | Standzeit (in Tagen) |
|---|---|
| 1 | 25 |
| 8 | 23 |
| 9 | 28 |
| 16 | 21 |
| 17 | 35 |
| ohne Stabilisator | 2 |
| handelsüblicher Stabilisator +) | 20 |

+) 3,3-Bis-(3'-tert.-butyl-4'-hydroxyphenyl)-butansäureethandioldiester

Wie aus der Tabelle zu entnehmen ist, sind die erfindungsgemässen Substanzen hervorragend zur Stabilisierung von Polyolefinen geeignet.

## Patentansprüche

1. Phenolgruppen enthaltende Epoxidharze und ihre Homooligomerisate der allgemeinen Formel (I)

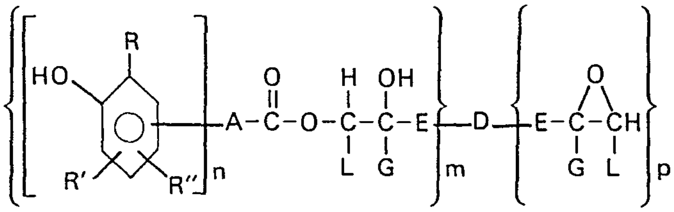

(I)

in der

R = $C_2$ bis $C_4$ Alkyl, vorzugsweise t-Butyl, ist,

R' und R'' unabhängig voneinander H oder $C_1$ bis $C_4$ Alkyl bedeuten,

n = 1 oder 2 sein soll und

A = eine chemische Bindung oder

α) der Rest eines geradkettigen oder verzweigten, unsubstituierten oder phenylsubstituierten Alkans mit 1 bis 20 C-Atomen, oder

β) der Rest eines unsubstituierten oder $C_1$ bis $C_5$ alkylsubstituierten, cycloaliphatischen Alkans mit 5 bis 12 C-Atomen, oder

γ) ein unsubstituierter oder $C_1$ bis $C_{12}$ alkylsubstituierter Phenyl- oder Naphthylrest ist,

E für eine $CH_2$-Gruppe,

G für ein H-Atom oder eine $CH_3$-Gruppe und

L für ein H-Atom steht, oder

E und L zusammen mit den sie verbindenden C-Atomen einen Cycloalkylrest mit 5 bis 12 C-Atomen, der auch alkylsubstituiert sein kann, bilden, und

D = Sauerstoff oder ein Radikal der Formel

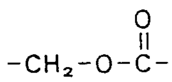

oder 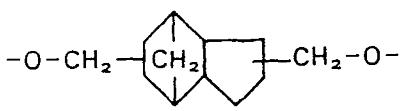

oder $-O-(CH_2)_q-O-[CH_2-CHOH-CH_2-O-(CH_2)_q-O]_r$,

oder 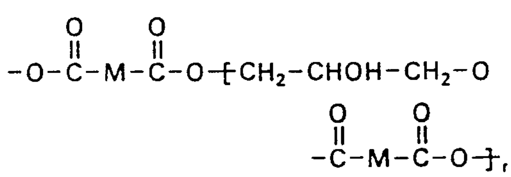

mit M = $-(CH_2)_{q-2}-$, 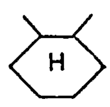 , 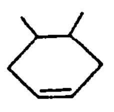 ,

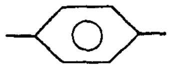 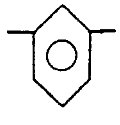 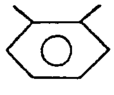

oder 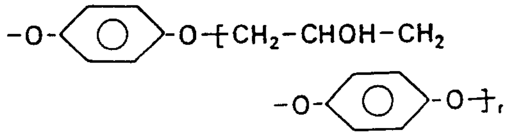

oder 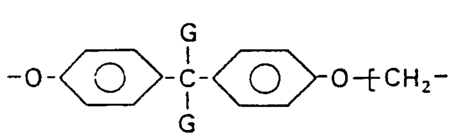

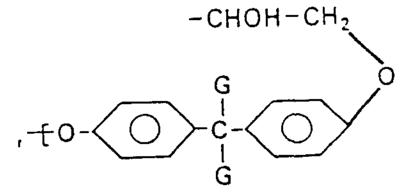

mit q = 2 bis 10 und r = 0 bis 10

oder 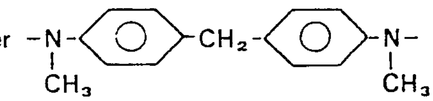

oder 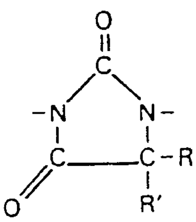

oder 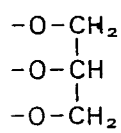 oder 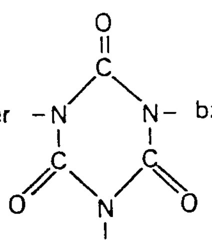 bzw. 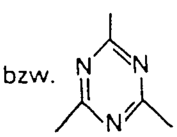

oder 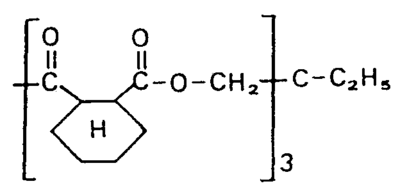

sein soll,

m = 1, 2 oder 3, und

p = 0, 1 oder 2 bedeutet, mit der Einschränkung, das p + m entweder 2 oder 3 ist.

2. Verwendung von Verbindungen nach Anspruch 1 zum Stabilisieren von Homo- und Copolymerisaten aus $C_2$ bis $C_4$ α-Olefinen gegen die schädigende Wirkung von Licht und Wärme in einer Menge von 0,001 bis 5,0 Gewichtsteilen auf 100 Gewichtsteile Polymerisat, gegebenenfalls im Gemisch mit weiteren Licht- und Wärmestabilisatoren.

3. Kunststoffmassen auf der Basis von Homo- und Copolymerisaten von α-Olefinen mit 2 bis 4 C-Atomen, in denen als Stabilisator gegen den schädigenden Einfluss von Wärme und Licht 0,001 bis 5,0 Gewichtsteile, bezogen auf 100 Gewichtsteile Polymerisat, einer Verbindung nach Anspruch 1 enthalten sind.

## Claims

1. Epoxide resins containing phenol groups and homo-oligomers thereof of the general formula (I):

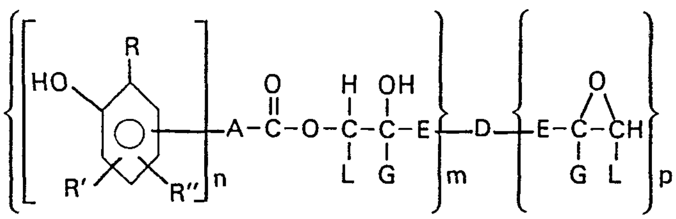

(I)

in which

R = $C_2$ to $C_4$ alkyl, preferred t-butyl,

R′ and R″ denote independently of one another H or $C_1$ to $C_4$ alkyl, n is 1 or 2 and A is a chemical bond or α) the radical of a straight-chain or branched, unsubstituted or phenyl-substituted alkane having 1 to 20 C atoms, or β) the radical of a cycloaliphatic alkane which has 5 to 12 C atoms and is unsubstituted or substituted by $C_1$ to $C_5$ alkyl, or γ) a phenyl or naphthyl radical unsubstituted or substituted by $C_1$ to $C_{12}$ alkyl, E represents a $CH_2$ group, G represents an H atom or a $CH_3$ group and L represents an H atom, or E and L, conjointly with the C atoms linking them, form a cycloalkyl radical which has 5 to 12 C atoms and which can also be alkyl-substituted, and D should be oxygen or a radical of the formula

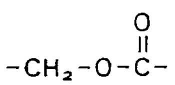

or 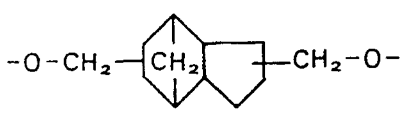

or $-O-(CH_2)_q-O-[CH_2-CHOH-CH_2-O-(CH_2)_q-O-]_r$

or 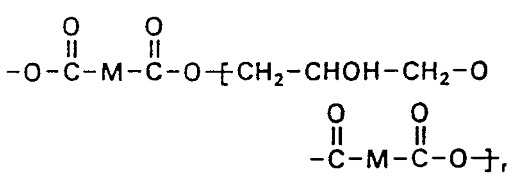

in which

M = $-(CH_2)_{q-2}$, 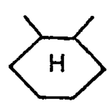 , 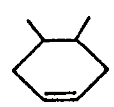 ,

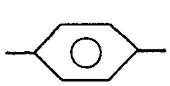 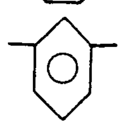 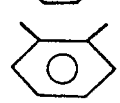

or 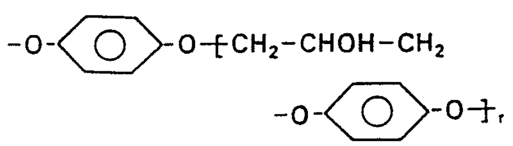

or 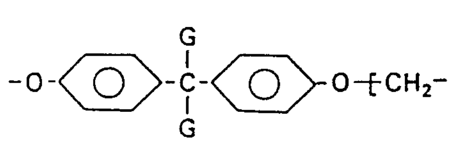

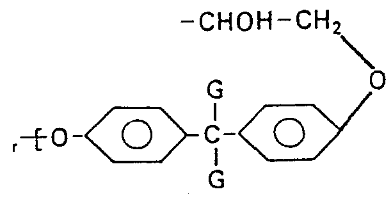

in which q = 2 to 10 and r = 0 to 10,

or 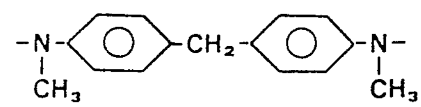

or 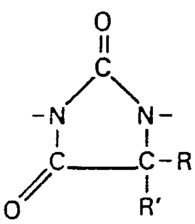

or 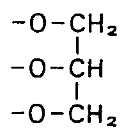 or 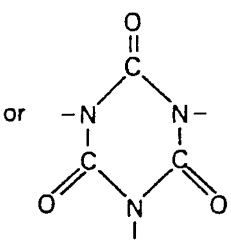 or 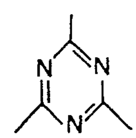

or 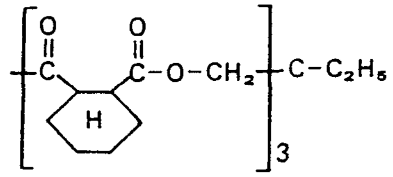

and m denotes 1, 2 or 3 and p denotes 0, 1 or 2, with the limitation that p+m is either 2 or 3.

2. Use of compounds according to claim 1 for stabilizing homopolymers and copolymers of α-olefins formed from $C_2$ to $C_4$ against the harmful action of light and heat, in a quantity of 0.001 to 5.0 parts by weight on 100 parts by weight of polymer, suitably besides further light stabilizers and heat stabilizers.

3. Plastic compositions based on homopolymers and copolymers of α-olefins having 2 to 4 C atoms, in which 0.001 to 5.0 parts by weight on 100 parts by weight of polymer of a compound according to claim 1 are present as stabilizer against the harmful influence of heat and light.

## Revendications

1. Résines époxydiques contenant des radicaux phénoliques et leur homooligomères qui répondent à la formule générale (I):

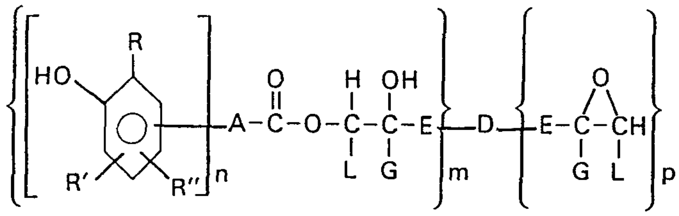
(I)

dans laquelle:

R représente un radical alkyle en $C_2$-$C_4$, de préférence un radical tert.-butyle;

R' et R'' représentent chacun, indépendamment l'un de l'autre, H ou un alkyle en $C_1$-$C_4$,

n est égal à 1 ou 2,

A représente une liaison chimique, ou

α) le radical d'un alcane linéaire ou ramifié, en $C_1$-$C_{20}$, non substitué ou porteur d'un phényle, ou

β) le radical d'un alcane cycloaliphatique en $C_5$-$C_{12}$, non substitué ou porteur d'un alkyle en $C_1$-$C_5$, ou

γ) un radical phényle ou naphtyle non substitué ou porteur d'un alkyle en $C_1$-$C_{12}$,

E représente un radical $-CH_2-$,

G représente un atome d'hydrogène ou un radical $-CH_3$ et

L représente un atome d'hydrogène, ou

E et L forment ensemble et avec les atomes de carbone qui les unissent un radical cycloalkyle contenant de 5 à 12 atomes de carbone, radical qui peut également porter un alkyle, et

D représente l'oxygène ou un radical de formule:

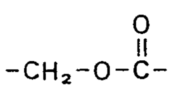

ou 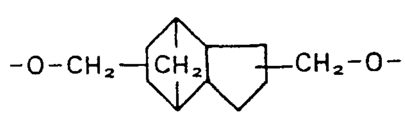

ou $-O-(CH_2)_q-O-[CH_2-CHOH-CH_2-O-(CH_2)_q-O]_r$

ou 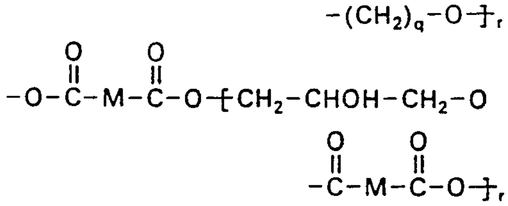

avec M = $-(CH_2)_{q-2}$, 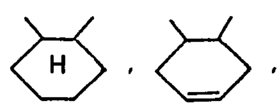 ,

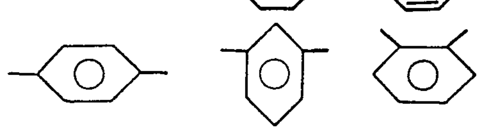

ou 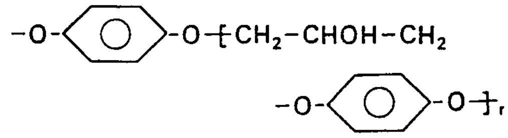

ou 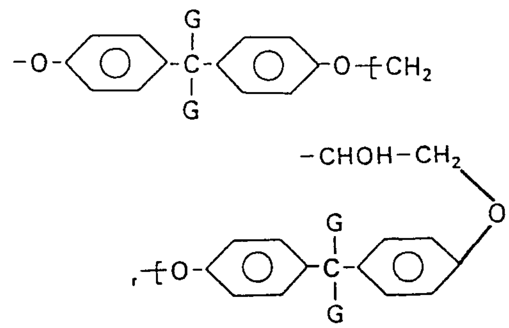

avec q = 2 à 10 et r = 0 à 10

ou 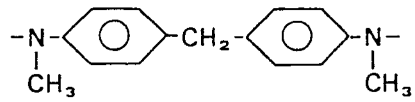

ou 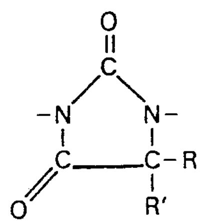

ou 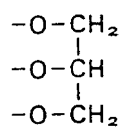 ou 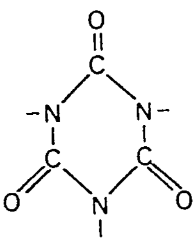 ou 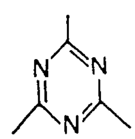

ou 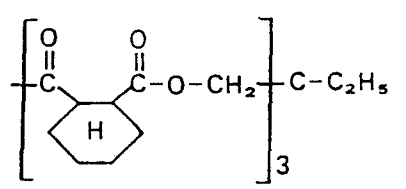

m est égal à 1, 2 ou 3, et

p est égal à 0, 1 ou 2, avec cette restriction que la somme (p+m) est égale à 2 ou à 3.

2. Application de composés selon la revendication 1 pour la stabilisation d'homopolymères et de copolymères d'α-oléfines en $C_2$-$C_4$ contre les effets destructeurs de la lumière et de la chaleur, en une quantité de 0,001 à 5,0 parties en poids pour

100 parties en poids du polymère, éventuellement en mélange avec d'autres stabilisants à la lumière et à la chaleur.

3. Matières plastiques à base d'homopolymères ou de copolymères d'α-oléfines en $C_2$-$C_4$, matières plastiques qui contiennent, comme stabilisant contre les effets destructeurs de la chaleur et de la lumière, de 0,001 à 5,0 parties en poids pour 100 parties en poids du polymère d'un composé selon la revendication 1.